(19) Europäisches Patentamt
European Patent Office
Office européen des brevets

(11) Publication number: **0 029 265**
**B1**

# (12) EUROPEAN PATENT SPECIFICATION

(45) Date of publication of patent specification: **17.08.83**

(21) Application number: **80201031.4**

(22) Date of filing: **30.10.80**

(51) Int. Cl.$^3$: **A 01 N 43/44,**
C 07 D 205/04

(54) **A method for preparing azetidine derivatives, male sterility in plants producing compositions containing them, their use in sterilizing male parts in plants, a method of producing F1 hybrid seed and certain novel azetidine derivatives.**

(30) Priority: **16.11.79 GB 7939781**

(43) Date of publication of application:
**27.05.81 Bulletin 81/21**

(45) Publication of the grant of the patent:
**17.08.83 Bulletin 83/33**

(84) Designated Contracting States:
**AT BE CH DE FR GB IT LI NL SE**

(56) References cited:
**CHEMICAL ABSTRACTS, vol. 66, no. 5, January 30, 1967, page 1642, abstract 17105y COLUMBUS, Ohio (US)**

**JOURNAL OF ORGANIC CHEMISTRY, vol. 37, no. 24, 1972 WASHINGTON D.C. (US)
A. G. ANDERSON et al.: "The synthesis of azetidine-3-carboxylic acid" pages 3953—3955**

**The file contains technical information submitted after the application was filed and not included in this specification**

(73) Proprietor: **SHELL INTERNATIONALE RESEARCH MAATSCHAPPIJ B.V.
Carel van Bylandtlaan 30
NL-2596 HR Den Haag (NL)**

(72) Inventor: **Devlin, Barry Roy John
59 Ruins Barn Road
Sittingbourne Kent (GB)**

(74) Representative: **Rogers, Roy Charles et al,
4 York Road
London SE1 7NA (GB)**

(56) References cited:
**JOURNAL OF THE AMERICAN CHEMICAL SOCIETY, vol. 91, no. 26, December 17, 1969 WASHINGTON D.C. (US)
K. ISONO et al.: "Studies on Polyoxins, Antifungal Antibiotics. XIII: The Structure of Polyoxins pages 7490—7505**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England

**EP 0 029 265 B1**

# A method for preparing azetidine derivatives, male sterility in plants producing compositions containing them, their use in sterilizing male parts in plants, a method of producing $F_1$ hybrid seed and certain novel azetidine derivatives

This invention relates to a method of sterilising male parts in plants, to compositions and compounds for use in such a method, and to $F_1$ hybrid seeds produced by such a method.

To obtain $F_1$ hybrid seeds, which have many advantages over non-hybrid seeds, seed-breeders cross-pollinate carefully selected parent plants. In the case of plants, for example small-grain cereal plants, which have hermaphroditic flowers and normally self-pollinate, this is achieved by removing the male anthers from each of the flowers by hand, an operation which is extremely time-consuming and requires highly-skilled workers. Much research is being carried out into treatments with chemicals by which this same result can be achieved.

Chemical Abstracts *66*(1967) 17105Y discloses that L-azetidine-2-carboxylic acid inhibits pollen germination *in vitro*.

The present invention provides a method of sterilising the male parts of a plant, which comprises applying to the plant an azetidine derivative which is an acid of the general formula I or a salt and/or an ester, amide, alkylamide, hydrazide or alkylhydrazide thereof:

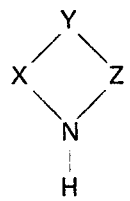

(I)

in which:

X represents one of the groups $CH_2$, CHR or $CR_2$;

Y represents one of the groups CHR, $CR_2$ or $CH.CO_2H$ and

Z represents one of the groups $CH_2$, CHR, $CR_2$ or $CH.CO_2H$;

the or each R independently represents an alkyl, alkenyl or cycloalkyl group or an aryl or aralkyl group optionally substituted on the aryl nucleus by one or more of the same or different substituents selected from halogen atoms, alkyl groups and alkoxy groups;

and one but only one of Y and Z must represent a $CH.CO_2H$ group.

Preferably the or each R independently represents an alkyl or alkenyl group having up to 6, especially up to 4, carbon atoms, a cycloalkyl group having from 3 to 6 carbon atoms, or a phenyl or benzyl group optionally substituted by one or more, preferably one or two, of the same or different substituents selected from chlorine, fluorine or bromine atoms, alkyl groups having from 1 to 4 carbon atoms and alkoxy groups having 1 to 4 carbon atoms.

More preferably the or each R independently represents a methyl or ethyl group or a phenyl group optionally substituted by one or two substituents selected from fluorine and chlorine atoms and methyl and methoxy groups.

Preferably X represents $CH_2$, Y represents CHR or $CH.CO_2H$ and Z represents $CH_2$ or $CH.CO_2H$, one of Y and Z being $CH.CO_2H$.

The azetidine derivative may for example be the free acid of the general formula I; a hydrohalide or an alkali metal salt thereof; the amide or hydrazide thereof in which the amide or hydrazide group may be substituted by one or two alkyl, preferably C(1—4) alkyl, especially methyl, groups; an alkyl, alkenyl or aralkyl ester, preferably an alkyl or alkenyl ester having up to 10, especially up to 7, carbon atoms in the alkyl or alkenyl group; or a hydrohalide of such an amide, hydrazide or ester.

Preferably the azetidine derivative is the free acid of formula I, a hydrohalide or an alkali metal salt thereof, the hydrazide thereof, a C(1—10) alkyl ester thereof or a hydrohalide of said hydrazide or ester.

Especially preferred azetidine derivatives are 3-carboxyazetidine and 2-carboxy-3-methylazetidine.

The azetidine derivative may exist in the form of isomers depending on the meaning of the groups X, Y and Z. For example, 2-carboxy-3-methylazetidine exists as geometric isomers depending on the relative positions of the carboxy and the methyl group, and in addition, for each of these geometric isomers, optical isomers exist. As is usual in processes involving biological systems, some isomers may be more active in the process of the invention than other isomers.

The process according to the invention produces plants in which male sterility has been produced without substantial effect on female fertility. Preferably the process is applied to cereal plants, for example maize and sorghum, especially to small-grain cereal plants such as wheat and barley, preferably when the plant is at a stage of growth between late tillering and emergence of the ear. The active compound is suitably applied at a dosage of from 0.05 to 2 kg/ha, preferably 0.25 to 1 kg/ha.

The present invention also provides a method of producing $F_1$ hybrid seed, which includes cross-pollinating a plant which has been treated by a process according to the invention with a second plant of a different strain.

The azetidine derivative is suitably formulated as a male sterility in plants producing composition for use in the process of the invention. The invention therefore also provides a male sterility in plants producing composition which comprises a compound of the general formula I or a salt and/or an ester, amide, alkylamide, hydrazide or alkylhydrazide thereof together with a suitable carrier.

Most of the azetidine derivatives are novel. The invention therefore also provides a novel azetidine derivative which is an acid of the formula I given above or a salt and/or an ester, amide, alkylamide, hydrazide or alkylhydrazide thereof, with the proviso that if Y is $CHC_2H_5$ or $CH.CO_2H$, then X must be CHR or $CR_2$.

A preferred novel azetidine derivative is 3-methyl-2-carboxyazetidine.

The invention also provides a process for the preparation of a novel compound according to the invention, which comprises either

(a) preparing an azetidine derivative in which Y represents a $CH.CO_2H$ group, by hydrolysing or alcoholising the corresponding cyano compound, in which compound the 1-nitrogen atom of the ring may be substituted by a group A which is a protecting group capable of being removed from the azetidine ring; and if required, removing the protecting group; or

(b) preparing an azetidine derivative in which Z represents a $CH.CO_2H$ group by cyclising a compound of the general formula

$$Hal\ \overset{\ominus\oplus}{N}H_3-X-Y-\underset{\underset{\displaystyle Hal}{|}}{CH}-CO_2H \qquad (II)$$

in which each Hal independently represents a chlorine or bromine atom and X and Y have the meanings given for the azetidine derivative, or by cyclising an ester of a compound of the general formula

$$Hal-X-Y-\underset{\underset{\displaystyle Hal}{|}}{CH}-CO_2H \qquad (III)$$

in which Hal, X and Y have the meanings given above, in the presence of an amine $ANH_2$ where A has the meaning given above, and subsequently removing the group A from the azetidine ring; and in the event of one azetidine derivative resulting from process (a) or (b) and a different azetidine derivative being required, converting the resulting derivative into the desired derivative.

Process (a) may be carried out using any known method for the conversion of a cyano group into a carboxyl group or derivative thereof. Hydrolysis may for example be carried out by treating the cyano compound with an aqueous acid or alkali, suitably under mild conditions, to produce an azetidine derivative in the form of the free acid or salt thereof. Alcoholysis may be carried out by refluxing the cyano compound in an alcohol in the presence of dry hydrogen chloride to form an azetidine derivative in the form of an ester or the hydrochloride salt thereof.

If a protecting group A is present, this group may be removed by any suitable method. Especially useful protecting groups A are the benzyl and, especially, $\alpha$-phenylbenzyl groups, which can be removed from the azetidine ring under mild conditions by reaction with gaseous hydrogen and a catalyst such as palladium or palladium hydroxide on charcoal.

The cyano compound may for example be prepared by reacting the corresponding hydroxy compound with methylsulphonyl chloride to form the 3-methylsulphonylazetidine analogue, and treating this compound with an alkali metal cyanide, suitably sodium cyanide. The 3-hydroxy compound may be prepared by reacting a halo epoxide with a primary amine.

In process (b), the compound of the general formula II may be cyclised by treatment with barium hydroxide, which, if the free acid of formula II is used, leads to the barium salt of an azetidine 2-carboxylic acid. Preferably, however, process (b) is carried out by cyclisation of an ester of a compound of the general formula III in the presence of an amine which is conveniently carried out by refluxing the two reactants together in a polar solvent, for example acetonitrile. Preferably the amine is benzylamine or $\alpha$-phenylbenzylamine, so that the benzyl or $\alpha$-phenylbenzyl group can be readily removed from the azetidine ring by hydrogenation as described for process (a) above.

The compound of the general formula II may be prepared using a modification of the Gabriel phthalimide synthesis. A compound of the general formula

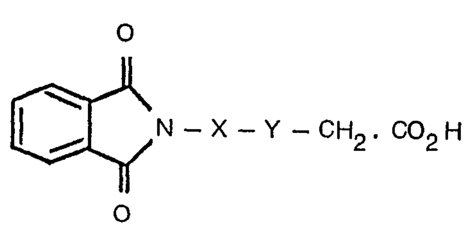

(IV)

may be halogenated, preferably using bromine and red phosphorus, to produce a compound of the general formula

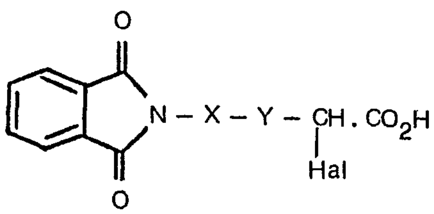

(V)

from which the desired amine hydrohalide can be released by treatment with a hydrohalic acid.

The compound of the general formula III may be prepared by reacting a lactone of the general formula

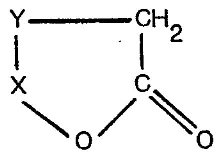

(VI)

with a halogen, preferably bromine, in the presence of red phosphorus, and reacting the product with an alcohol in the presence of an acid catalyst. In a preferred embodiment of the process the alcohol used is benzyl alcohol. It is then possible to react the benzyl ester of the compound of formula III with benzylamine or α-phenylbenzylamine to produce a compound of formula

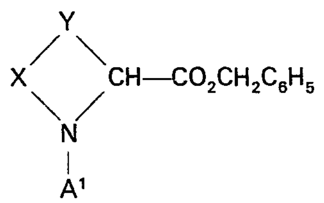

(VII)

where $A^1$ is benzyl or α-phenylbenzyl.

Subsequent catalytic hydrogenation cleave both the benzyl ester group and the N—$A^1$ group giving the free acid of formula I.

A carrier in a composition according to the invention is any material with which the active ingredient is formulated to facilitate application to the plant to be treated, or to facilitate storage, transport or handling. A carrier may be a solid or a liquid, including a material which is normally gaseous but which has been compressed to form a liquid, and any of the carriers normally used in formulating agricultural compositions may be used.

Suitable solid carriers include natural and synthetic clays and silicates, for example natural silicas such as diatomaceous earths; magnesium silicates, for example talcs; magnesium aluminium silicates, for example attapulgites and vermiculites; aluminium silicates, for example kaolinites, montmorillonites and micas; calacium carbonate; calcium sulphate; synthetic hydrated silicon oxides and synthetic calcium or aluminium silicates; elements, for example carbon and sulphur; natural and synthetic resins, for example coumarone resins, polyvinyl chloride, and styrene polymers and copolymers; solid polychlorophenols; bitumen; waxes, for example beeswax, paraffin wax, and chlorinated mineral waxes; and solid fertilisers, for example superphosphates.

Suitable liquid carriers include water; alcohols, for example isopropanol and glycols; ketones, for example acetone, methyl ethyl ketone, methyl isobutyl ketone and cyclohexanone; ethers; aromatic or araliphatic hydrocarbons, for example benzene, toluene and xylene; petroleum fractions, for example kerosine and light mineral oils; chlorinated hydrocarbons, for example carbon tetrachloride, perchloroethylene and trichloroethane. Mixtures of different liquids are often suitable.

Agricultural compositions are often formulated and transported in a concentrated form which is subsequently diluted by the user before application. The presence of small amounts of a carrier which is a surface-active agent facilitates this process of dilution. Thus preferably at least one carrier in a composition according to the invention is a surface-active agent. For example the composition may contain at least two carriers, at least one of which is a surface-active agent.

A surface-active agent may be an emulsifying agent, a dispersing agent or a wetting agent; it may be nonionic or ionic. Examples of suitable surface-active agents include the sodium or calcium salts of polyacrylic acids and lignin sulphonic acids; the condensation products of fatty acids or aliphatic amines or amides containing at least 12 carbon atoms in the molecule with ethylene oxide and/or propylene oxide; fatty acid esters of glycerol, sorbitan, sucrose or pentaerythritol; condensates of these with

ethylene oxide and/or propylene oxide; condensation products of fatty alcohol or alkyl phenols, for example *p*-octylphenol or *p*-octylcresol, with ethylene oxide and/or propylene oxide; sulphates or sulphonates of these condensation products; alkali or alkaline earth metal salts, preferably sodium salts, of sulphuric or sulphonic acid esters containing at least 10 carbon atoms in the molecule, for example sodium lauryl sulphate, sodium secondary alkyl sulphates, sodium salts of sulphonated castor oil, and sodium alkylaryl sulphonates such as sodium dodecylbenzene sulphonate; and polymers of ethylene oxide and copolymers of ethylene oxide and propylene oxide.

The compositions of the invention may for example be formulated as wettable powders, dusts, granules, solutions, emulsifiable concentrates, emulsions, suspension concentrates and aerosols. Wettable powders usually contain 25, 50 and 75% w of active ingredient and usually contain, in addition to solid inert carrier, 3—10% w of a dispersing agent and, where necessary, 0—10% w of stabiliser(s) and/or other additives such as penetrants or stickers. Dusts are usually formulated as a dust concentrate having a similar composition to that of a wettable powder but without a dispersant, and are diluted in the field with further solid carrier to give a composition usually containing $\frac{1}{2}$—10% w of active ingredient. Granules are usually prepared to have a size between 10 and 100 BS mesh (1.676—0.152 mm), and may be manufactured by agglomeration or impregnation techniques. Generally, granules will contain $\frac{1}{2}$—25% w active ingredient and 0—10% w of additives such as stabilisers, slow release modifiers and binding agents. Emulsifiable concentrates usually contain, in addition to a solvent and, when necessary, co-solvent, 10—50% w/v active ingredient, 2—20% w/v emulsifiers and 0—20% w/v of other additives such as stabilisers, penetrants and corrosion inhibitors. Suspension concentrates are usually compounded so as to obtain a stable, non-sedimenting flowable product and usually contain 10—75% w active ingredient, 0.5—15% w of dispersing agents, 0.1—10% w of suspending agents such as protective colloids and thixotropic agents, 0—10% w of other additives such as defoamers, corrosion inhibitors, stabilisers, penetrants and stickers, and water or an organic liquid in which the active ingredient is substantially insoluble; certain organic solids or inorganic salts may be present dissolved in the formulation to assist in preventing sedimentation or as anti-freeze agents for water.

Aqueous dispersions and emulsions, for example compositions obtained by diluting a wettable powder or a concentrate according to the invention with water, also lie within the scope of the present invention. The said emulsions may be of the water-in-oil or of the oil-in-water type, and may have a thick 'mayonnaise'-like consistency.

The compositions of the invention may also contain other ingredients, for example, other compounds possessing insecticidal, herbicidal or fungicidal properties.

The following Examples illustrate the invention.

## Example 1

Preparation of 3-methyl-2-carboxyazetidine

(a) 4-methyltetrahydrofuran-2-one (6.5 g, 0.065 mol) and red phosphorus (0.4 g) were stirred together and heated to between 100 and 115°C. The mixture was treated with bromine (30 g, 0.18 mol) until hydrogen bromide began to be evolved. The reaction mixture was then cooled to between 0 and 5°C, benzyl alcohol (36 g, 0.3 mol) was added, and the mixture was saturated with dry hydrogen chloride. The resulting mixture was allowed to stand for 24 hours. Diethyl ether (100 ml) was added, and the mixture was poured into a 3% aqueous solution of sodium bicarbonate, containing ice. The mixture was extracted several times with diethyl ether. The ether extracts were dried over magnesium sulphate, and evaporated. The resulting pale yellow oil was subjected to vacuum distillation: all liquids boiling at a temperature of up to 122°C at a pressure 0.6 mmHg were removed. The residue (15 g) was shown by NMR to be the benzyl ester of 1,3-dibromo-2-methylbutanoic acid, obtained in 66% yield.

| *Analysis* | C | H | Br |
|---|---|---|---|
| Calculated for $C_{12}H_4Br_2O_2$ | 41.1 | 4.0 | 45.7 |
| Found | 44.1 | 4.5 | 44.6 |

(b) The dibromo ester obtained in (a) (15.0 g, 0.043 mol), $\alpha$-phenylbenzylamine (27.8 g, 0.13 mol) and acetonitrile (250 ml) were refluxed together for 24 hours. The resulting mixture was filtered, evaporated to dryness, triturated with diethyl ether (200 ml), and filtered again. Dry hydrogen chloride was then passed into the ether solution for 5 minutes. The resulting off-white solid was collected, suspended in chloroform (300 ml) and treated with a slight excess of triethylamine (20 ml). The resulting clear solution was evaporated to dryness and extracted with diethyl ether. The resulting ether solution was evaporated, giving 10 g of an oil which was passed over a silica column using as eluant a mixture of ethyl acetate and petroleum ether (boiling point 60—80°C) in a ratio 1.5:8.5. Evaporation of the fast-running fractions gave an oil which solidified on standing. The solid was recrystallised from ethanol to give 2.2 g of the benzyl ester of 1-diphenylmethyl-2-carboxy-3-methylazetidine, melting point 101—102°C.

| *Analysis* | C | H | N |
|---|---|---|---|
| Calculated for $C_{25}H_{25}NO_2$ | 80.8 | 6.8 | 3.8 |
| Found | 80.2 | 6.8 | 3.6 |

(c) The cyclic ester in (b) (2.0 g, 0.0054 mol) was suspended in ethanol (500 ml) and methanol (20 ml), a 5% palladium/charcoal catalyst (0.5 g) was added and the mixture was hydrogenated in a Parr apparatus under a hydrogen pressure of between 3 and 4 atmospheres absolute, for 15 hours. The mixture was then filtered and evaporated. Diethyl ether was added, and the aqueous phase was extracted several times and then evaporated to dryness leaving 0.5 g of a white solid, which was recrystallised from ethanol to give 2-carboxy-3-methylazetidine, melting point 200—201°C with decomposition, in 83% yield.

| *Analysis* | C | H | N |
|---|---|---|---|
| Calculated for $C_5H_9NO_2$ | 52.17 | 7.8 | 12.17 |
| Found | 51.2 | 8.2 | 11.9 |

NMR showed that the compound was a mixture of geometric isomers in the ratio 9:1.

## Example 2

Preparation of 3-carboxyazetidine

(a) 1-diphenylmethyl-3-cyanoazetidine (15 g, 0.06 mol) was mixed with 2-methoxyethanol (150 ml) and added to a solution of potassium hydroxide (13 g, 0.2 mol) in water (10 ml). The resulting mixture was refluxed for 15 hours, during which time ammonia was evolved, and then poured onto ice and water (1500 ml). This mixture was then acidified with dilute hydrochloric acid until the pH was 1.5, and was then extracted with dichloromethane. The aqueous phase was adjusted to pH 5 by addition of solid sodium bicarbonate to the stirred solution.

1-diphenylmethyl-3-carboxyazetidine separated from the solution as a fine white solid, over about half an hour. The solid was collected, washed with water and air-dried. 13 g was obtained, corresponding to a yield of 81%. Melting point: 190—191°C with decomposition.

| *Analysis* | C | H | N |
|---|---|---|---|
| Calculated for $C_{17}H_{17}NO_2$ | 76.4 | 6.4 | 5.2 |
| Found | 75.4 | 6.4 | 5.1 |

(b) The N-substituted acid obtained in (a) (10 g, 0.037 mol) was suspended in methanol (300 ml), and 6 g of a catalyst of palladium II hydroxide on charcoal, prepared by the method described in Tetrahedron Letters, 1967, page 1663, were added. The mixture was hydrogenated in a Parr apparatus at a hydrogen pressure of about $3\frac{1}{2}$ atmospheres absolute for 3 hours. Uptake of hydrogen stopped abruptly after 1 hour. The solution was filtered and evaporated almost to dryness under reduced pressure. The aqueous residue was extracted several times with dichloromethane and then evaporated to dryness. The white solid residue was recrystallised from ethanol to give 2.6 g of shiny plates of hydrated 3-carboxyazetidine, melting point 169—170°C with decomposition.

| *Analysis* | C | H | N |
|---|---|---|---|
| Calculated for $C_4H_7NO_2.H_2O$ | 40.3 | 7.5 | 11.7 |
| Found | 40.3 | 6.7 | 11.6 |

Evaporation of the recrystallising solvent gave a further crop of 1.2 g of crystals, melting point 235—270°C with decomposition. These crystals consisted of anhydrous 3-carboxyazetidine, and had an NMR spectrum identical to the hydrated acid. The overall yield of step (b) was 95%.

## Example 3

Preparation of 3-ethyl-2-carboxy azetidine

(a) Bromine (21 g, 0.132 mol) was added to a stirred mixture of 4-ethyl-γ-butyro-lactone (15 g, 0.132 mol) and red phosphorus (0.3 g) maintained at 120°C. The bromine was added just below the surface of the reactant mixture and during the final stages of bromine addition, the evolution of hydrogen bromide was observed. The reaction mixture was then cooled, benzyl alcohol (40 ml) added and the resulting solution was saturated with dry hydrogen chloride gas at ambient temperatures. The resulting mixture was allowed to stand for 24 hours. The mixture was then partitioned between ether and a sodium bicarbonate solution and the ether layer was separated and dried over magnesium sulphate. Distillation of the dried solution afforded the benzyl ester of 2-bromo-3-bromomethyl pentanoic acid (b.p. 149°C/0.7 mm) in a 43% yield.

| *Analysis* | C | H | Br |
|---|---|---|---|
| Calculated $C_{13}H_{10}Br_2O_2$ | 42.9 | 4.4 | 44.0 |
| Found | 43.5 | 4.5 | 43.3 |

(b) The dibromo ester obtained in (a) (19.5 g, 0.054 mol) and benzhydrylamine (29 g, 0.16 mol) in acetonitrile were stirred and refluxed for 48 hours. The resulting mixture was cooled, filtered free of insoluble amine salt and the filtrate evaporated to dryness. The residue, which was a complex mixture of product, was partially purified using a column of silica-gel and petroleum ether/ethyl acetate as the eluent. The first fast-running products to emerge were collected and evaporated to dryness, leaving the benzyl ester of 1-diphenylmethyl-2 carboxy-3-ethylazetidine as the major component (65% by NMR) of the crude residual mixture (8 g).

(c) A mixture of the crude ester obtained in (b) (8 g) ethanol (100 ml) and 5% palladium charcoal catalyst (10 g) was hydrogenated at ambient temperature at a hydrogen pressure of between 3 and 4 atmospheres absolute for 10 hours in a Parr apparatus. The resulting mixture was filtered, evaporated to dryness and partitioned between methylene chloride and water. The aqueous phase was separated, evaporated to a low bulk and poured onto a Dowex 8—50 x(H) resin column. Isolation of the imino acid was achieved by eluting the washed column with a 2M ammonia solution. Evaporation of the washings left a white solid which on recrystallization from ethanol deposited an isomer mixture [1$\alpha$:3$\beta$] of 3-ethyl-2-carboxyazetidine.

| *Analysis* | C | H | N |
|---|---|---|---|
| Calculated for $C_6H_{11}NO_2$ | 55.8 | 8.5 | 10.85 |
| Found | 55.8 | 8.8 | 10.7 |

Evaporation of the recrystallizing medium left additional 3-ethyl-2-carboxyazetidine having a different isomer composition [2$\alpha$:3$\beta$]

| *Analysis* | C | H | N |
|---|---|---|---|
| Found | 55.8 | 8.9 | 10.7 |

Example 4

Demonstration of male sterility in plants producing activity

Spring wheat, variety Sicco, was propagated in a glass-house in 13 cm pots containing a loam-based compost. Supplementary lighting was provided by high-pressure mercury vapour lamps to give a constant day length of 16 hours. The temperature was maintained at approximately 20°C.

The compound to be tested was formulated as an aqueous solution containing 0.1% nonidet P 40 (trade mark) as wetting agent and 1% acetone to aid solubility. This formulation was diluted with water to a concentration of 1000 ppm, and sprayed onto plants to run-off. The plants were treated at the growth stage when the second node of the plant was just detectable.

At ear emergence but before anthesis, 5 heads from each treated pot were placed in cellophane bags to prevent cross-pollination. At maturity, the bagged ears were harvested, and seed set was recorded and compared with untreated controls.

The results are shown in the following Table.

TABLE

| Compound of Example No. | Grain Set Inhibition (% of control) |
|---|---|
| 1 | 69 |
| 2 | 87 |
| 3 [2$\alpha$:3$\beta$] | 68 |

It can be seen that the test compounds produced a considerable reduction in seed set compared with the untreated control, clearly illustrating the ability of the compounds to sterilise the male parts of the wheat.

**Claims for the Contracting States: BE CH DE FR GB IT NL SE**

1. A method of sterilising the male parts of a plant, characterized in that it comprises applying to the plant an azetidine derivative which is an acid of the general formula I or a salt and/or an ester, amide, alkylamide, hydrazide or alkylhydrazide thereof:

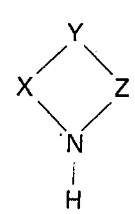

(I)

in which:

X represents one of the groups $CH_2$, CHR or $CR_2$;

Y represents one of the groups CHR, $CR_2$ or $CH.CO_2H$;

Z represents one of the groups $CH_2$, CHR, $CR_2$ or $CH.CO_2H$;

the or each R independently represents an alkyl, alkenyl or cycloalkyl group or an aryl or aralkyl group optionally substituted on the aryl nucleus by one or more of the same or different substituents selected from halogen atoms, alkyl groups and alkoxy groups;

and one but only one of Y and Z must represent a $CH.CO_2H$ group.

2. A method as claimed in Claim 1, in which the or each R independently represents an alkyl or alkenyl group having up to 6 carbon atoms, a cycloalkyl group having from 3 to 6 carbon atoms, or a phenyl or benzyl group optionally substituted by one or more of the same or different substituents selected from chlorine, bromine or fluorine atoms, alkyl groups having from 1 to 4 carbon atoms or alkoxy groups having from 1 to 4 carbon atoms.

3. A method as claimed in Claim 2, in which the or each R independently represents a methyl or ethyl group or a phenyl group optionally substituted by one or two substituents selected from fluorine and chlorine atoms and methyl and methoxy groups.

4. A method as claimed in any one of Claims 1 to 3, in which X represents $CH_2$, Y represents CHR or $CH.CO_2H$ and Z represents $CH_2$ or $CH.CO_2H$.

5. A method as claimed in any one of Claims 1 to 4, in which the azetidine derivative is the free acid of the general formula I or a hydrohalide or an alkali metal salt thereof; the amide or hydrazide thereof in which the amide or hydrazide group may be substituted by one or two alkyl groups; an alkyl, alkenyl or aralkyl ester; or a hydrohalide of said amide, hydrazide or ester.

6. A method as claimed in Claim 5, in which the azetidine derivative is the free acid of formula I, a hydrohalide or alkali metal salt thereof, the hydrazide thereof, a C(1—10) alkyl ester thereof or a hydrohalide of said hydrazide or ester.

7. A method as claimed in Claim 1, in which the azetidine derivative is 3-carboxyazetidine or 2-carboxy-3-methylazetidine.

8. A method of producing $F_1$ hybrid seed, which includes cross-pollinating a plant which has been treated by a method as claimed in any one of Claims 1 to 7, with a second plant of a different strain.

9. A male sterility in plants producing composition which comprises an azetidine derivative as defined in any one of Claims 1 to 7, together with a suitable carrier; provided that, if the azetidine derivative is 3-ethyl-2-carboxyazetidine and only one carrier is present, that carrier is not ethanol or water, if the azetidine derivative is 3-carboxyazetidine, and only one carrier is present, that carrier is not water and if the azetidine derivative is 3-carboxyazetidine and only two carriers are present, those carriers are not ethanol and water.

10. A composition as claimed in Claim 9, which comprises at least two carriers, at least one of which is a surface-active agent.

11. An azetidine derivative as defined in any one of Claims 1 to 3, with the proviso that if Y represents $CH.C_2H_5$ or $CH.CO_2H$, then X must represent a group CHR or $CR_2$.

12. 3-Methyl-2-carboxyazetidine.

13. A process for the preparation of a compound as claimed in Claim 11, which comprises either

(a) preparing an azetidine derivative in which Y represents a $CH.CO_2H$ group, by hydrolysing or alcoholising the corresponding cyano compound, in which compound the 1-nitrogen atom of the ring may be substituted by a group A which is a protecting group capable of being removed from the azetidine ring; and if required, removing the protecting group; or

(b) preparing an azetidine derivative in which Z represents a $CH.CO_2H$ group by cyclising a compound of the general formula

$$\overset{\ominus\oplus}{\mathrm{Hal\ NH_3}}\text{—X—Y—}\underset{\mathrm{Hal}}{\underset{|}{\mathrm{CH}}}\text{—}CO_2H$$

in which each Hal independently represents a chlorine or bromine atom and X and Y have the meanings given for the azetidine derivative, or by cyclising an ester of a compound of the general formula

$$\mathrm{Hal}\text{—X—Y—}\underset{\mathrm{Hal}}{\underset{|}{\mathrm{CH}}}\text{—}CO_2H \qquad \text{(III)}$$

in which Hal, X and Y have the meanings given above, in the presence of an amine $ANH_2$ where A has the meaning given above, and subsequently removing the group A from the azetidine ring; and in the event of one azetidine derivative resulting from process (a) or (b) and a different azetidine derivative being required, converting the resulting derivative into the desired derivative.

14. A process as claimed in Claim 13, in which in process (a) a protecting group A is present which is a benzyl or an $\alpha$-phenylbenzyl group, which group is removed from the azetidine ring after hydrolysis or alcoholysis of the cyano group, by hydrogenation using gaseous hydrogen and a catalyst.

15. A process as claimed in Claim 13, in which in process (b) a compound of the formula II is cyclised by treatment with barium hydroxide.

16. A process as claimed in Claim 13, in which in process (b) a compound of the formula III is cyclised in the presence of benzylamine or $\alpha$-phenylbenzylamine.

17. A compound as claimed in Claim 11, whenever prepared by a process as claimed in any one of Claims 13 to 16.

**Claims for the Contracting State: AT**

1. A method of sterilising the male parts of a plant, characterised in that it comprises applying to the plant an azetidine derivative which is an acid of the general formula I or a salt and/or an ester, amide, alkylamide, hydrazide or alkylhydrazide thereof:—

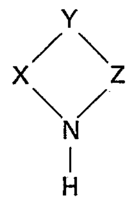

(I)

in which

X represents one of the groups $CH_2$, CHR or $CR_2$;

Y represents one of the groups CHR, $CR_2$ or $CH.CO_2H$;

Z represents one of the groups $CH_2$, CHR, $CR_2$, or $CH.CO_2H$;

the or each R independently represents an alkyl, alkenyl or cycloalkyl group or an aryl or aralkyl group optionally substituted on the aryl nucleus by one or more of the same or different substituents selected from halogen atoms, alkyl groups and alkoxy groups;

and one but only one of Y and Z must represent a $CH.CO_2H$ group.

2. A method as claimed in Claim 1, in which the or each R independently represents an alkyl or alkenyl group having up to 6 carbon atoms, a cycloalkyl group having from 3 to 6 carbon atoms, or a phenyl or benzyl group optionally substituted by one or more of the same or different substituents selected from chlorine, bromine or fluorine atoms, alkyl groups having from 1 to 4 carbon atoms or alkoxy groups having from 1 to 4 carbon atoms.

3. A method as claimed in Claim 2, in which the or each R independently represents a methyl or ethyl group or a phenyl group optionally substituted by one or two substituents selected from fluorine and chlorine atoms and methyl and methoxy groups.

4. A method as claimed in any one of Claims 1 to 3, in which X represents $CH_2$, Y represents CHR or $CH.CO_2H$ and Z represents $CH_2$ or $CH.CO_2H$.

5. A method as claimed in any one of Claims 1 to 4, in which the azetidine derivative is the free acid of the general formula I or a hydrohalide or an alkali metal salt thereof; the amide or hydrazide thereof in which the amide or hydrazide group may be substituted by one or two alkyl groups; an alkyl, alkenyl or aralkyl ester; or a hydrohalide of said amide, hydrazide or ester.

6. A method as claimed in Claim 5, in which the azetidine derivative is the free acid of formula I, a hydrohalide or alkali metal salt thereof, the hydrazide thereof, a C(1—10) alkyl ester thereof or a hydrohalide of said hydrazide or ester.

7. A method as claimed in Claim 1, in which the azetidine derivative is 3-carboxyazetidine or 2-carboxy-3-methylazetidine.

8. A method of producing $F_1$ hybrid seed, which includes cross-pollinating a plant which has been treated by a method as claimed in any one of Claims 1 to 7, with a second plant of a different strain.

9. A male sterility in plants producing composition which comprises an azetidine derivative as defined in any one of Claims 1 to 7, together with a suitable carrier; provided that, if the azetidine derivative is 3-ethyl-2-carboxy azetidine and only one carrier is present, that carrier is not ethanol or water, if the azetidine derivative is 3-carboxy azetidine and only one carrier is present, that carrier is not water and if the azetidine derivative is 3-carboxy azetidine and only two carriers are present, those carriers are not ethanol and water.

10. A composition as claimed in Claim 9, which comprises at least two carriers, at least one of which is a surface-active agent.

11. A process for the preparation of a compound as defined in Claim 1, wherein X represents a group CHR or $CR_2$ if Y represents $CH.C_2H_5$ or $CH.CO_2H$, which comprises either

(a) preparing an azetidine derivative in which Y represents a $CH.CO_2H$ group, by hydrolysing or alcoholising the corresponding cyano compound, in which compound the 1-nitrogen atom of the ring

may be substituted by a group A which is a protecting group capable of being removed from the azetidine ring; and if required, removing the protecting group; or

(b) preparing an azetidine derivative in which Z represents a $CH.CO_2H$ group by cyclising a compound of the general formula:—

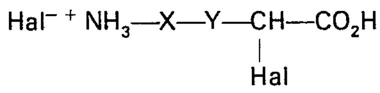

(II)

in which each Hal independently represents a chlorine or bromine atom and X and Y have the meanings given for the azetidine derivative, or by cyclising an ester of a compound of the general formula:—

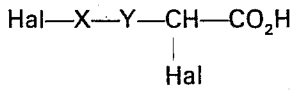

(III)

in which Hal, X and Y have the meanings given above, in the presence of an amine $ANH_2$ where A has the meaning given above, and subsequently removing the group A from the azetidine ring; and in the event of one azetidine derivative resulting from process (a) or (b) and a different azetidine derivative being required, converting the resulting derivative into the desired derivative.

12. A process as claimed in Claim 11, in which in process (a) a protecting group A is present which is a benzyl or an $\alpha$-phenylbenzyl group, which group is removed from the azetidine ring after hydrolysis or alcoholysis of the cyano group, by hydrogenation using gaseous hydrogen and a catalyst.

13. A process as claimed in Claim 11, in which in process (b) a compound of the formula II is cyclised by treatment with barium hydroxide.

14. A process as claimed in Claim 11, in which in process (b) a compound of the formula III is cyclised in the presence of benzylamine or $\alpha$-phenylbenzylamine.

**Patentansprüche für die Vertragsstaaten: BE CH DE FR GB IT NL SE**

1. Ein Verfahren zur Sterilisation der männlichen Teile einer Pflanze, dadurch gekennzeichnet, daß es das Aufbringen eines Azetidinderivats auf die Pflanze umfaßt, welches Derivat eine Säure der allgemeinen Formel (I) oder ein Salz und/oder ein Ester, Amid, Alkylamid, Hydrazid oder Alkylhydrazid hievon ist:

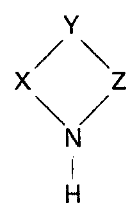

, (I)

worin

X eine der Gruppen $CH_2$, CHR oder $CR_2$ bedeutet;

Y eine der Gruppen CHR, $CR_2$ oder $CH.CO_2H$ bedeutet und

Z eine der Gruppen $CH_2$, CHR, $CR_2$ oder $CH.CO_2H$ darstellt;

der oder jeder Rest R unabhängig voneinander eine Alkyl-, Alkenyl- oder Cycloalkylgruppe oder eine Aryl- oder Aralkylgruppe bedeutet, die gewünschtenfalls am Arylkern durch eine oder mehrere gleiche oder verschiedene Substituenten, ausgewählt unter Halogenatomen, Alkylgruppen und Alkoxygruppen, substituiert sind;

und eines, aber nur eines der Symbole Y und Z eine $CH.CO_2H$-Gruppe darstellen muß.

2. Ein Verfahren nach Anspruch 1, worin der oder jeder Rest R unabhängig voneinander eine Alkyl- oder Alkenylgruppe mit bis zu 6 Kohlenstoffatomen, eine Cycloalkylgruppe mit 3 bis 6 Kohlenstoffatomen oder eine Phenyl- oder Benzylgruppe bedeutet, die gegebenenfalls durch eine oder mehrere gleiche oder verschiedene Substituenten, ausgewählt unter Chlor-, Brom- oder Fluoratomen, Alkylgruppen mit 1 bis 4 Kohlenstoffatomen oder Alkoxygruppen mit 1 bis 4 Kohlenstoffatomen, substituiert sind.

3. Ein Verfahren nach Anspruch 2, worin der oder jeder Rest R unabhängig voneinander eine Methyl- oder Ethylgruppe oder eine Phenylgruppe darstellt, die gegebenenfalls durch einen oder zwei Substituenten, ausgewählt unter Fluor- und Chloratomen und Methyl- und Methoxygruppen, substituiert ist.

4. Ein Verfahren nach einem der Ansprüche 1 bis 3, worin X für $CH_2$, Y für CHR oder $CH.CO_2H$ und Z für $CH_2$ oder $CH.CO_2H$ stehen.

5. Ein Verfahren nach einem der Ansprüche 1 bis 4, worin das Azetidinderivat die freie Säure der

allgemeinen Formel (I) oder ein Hydrohalogenid- oder ein Alkalimetallsalz hievon ist; das Amid oder Hydrazid hievon ist, worin die Amid- oder Hydrazidgruppe durch ein oder zwei Alkylgruppen substituiert sein kann; ein Alkyl-, Alkenyl- oder Aralkylester ist; oder ein Hydrohalogenid des genannten Amids, Hydrazids oder Esters ist.

6. Ein Verfahren nach Anspruch 5, worin das Azetidinderivat die freie Säure der Formel (I), ein Hydrohalogenid oder ein Alkalimetallsalz hievon, das Hydrazid hievon, ein $C_{1-10}$-alkylester hievon oder ein Hydrohalogenid des genannten Hydrazids oder Esters ist.

7. Ein Verfahren nach Anspruch 1, worin das Azetidinderivat 3-Carboxyazetidin oder 2-Carboxy-3-methylazetidin ist.

8. Ein Verfahren zur Herstellung von $F_1$-Hybridsamen, welches ein kreuzweises Befruchten einer Pflanze, die nach einem Verfahren gemäß einem der Ansprüche 1 bis 7 behandelt worden ist, mit einer zweiten Pflanze eines unterschiedlichen Stammes umfaßt.

9. Eine männliche Sterilität in Pflanzen hervorrufende Zusammensetzung, welche ein Azetidinderivat gemäß Definition in einem der Ansprüche 1 bis 7, zusammen mit einem geeigneten Träger, umfaßt, mit der Maßgabe, daß dann, wenn das Azetidinderivat 3-Ethyl-2-carboxyazetidin ist und nur ein Träger vorliegt, dieser Träger nicht Ethanol oder Wasser ist, daß dann, wenn das Azetidinderivat 3-Carboxyazetidin ist und nur ein Träger vorliegt, dieser Träger nicht Wasser ist und daß dann, wenn das Azetidinderivat 3-Carboxyazetidin ist und nur zwei Träger vorliegen, diese Träger nicht Ethanol und Wasser sind.

10. Eine Zusammensetzung nach Anspruch 9, welche wenigstens zwei Träger umfaßt, wovon wenigstens einer ein oberflächenaktives Mittel ist.

11. Ein Azetidinderivat gemäß Definition in einem der Ansprüche 1 bis 3, mit der Maßgabe, daß dann, wenn Y für $CH.C_2H_5$ oder $CH.CO_2H$ steht, X eine Gruppe CHR oder $CR_2$ bedeuten muß.

12. 3-Methyl-2-carboxyazetidin.

13. Ein Verfahren zur Herstellung einer Verbindung nach Anspruch 11, welches umfaßt entweder

(a) Bereitung eines Azetidinderivates, worin Y eine $CH.CO_2H$-Gruppe bedeutet, durch Hydrolyse oder Alkoholyse der entsprechenden Cyanoverbindung, in welcher Verbindung das 1-Stickstoffatom des Ringes durch eine Gruppe A substituiert sein kann, welche eine Schutzgruppe ist, die von dem Azetidinring entfernt werden kann; und, falls erforderlich, Abtrennung der Schutzgruppe; oder

(b) Bereitung eines Azetidinderivats, in welchem Z eine $CH.CO_2H$-Gruppe darstellt, durch Cyclisieren einer Verbindung der allgemeinen Formel

$$\overset{(-)}{Hal}\ \overset{(+)}{N}H_3—X—Y—\underset{\displaystyle Hal}{\underset{|}{CH}}—CO_2H \qquad , (II)$$

worin jeder Rest Hal unabhängig voneinander ein Chlor- oder Bromatom darstellt und X und Y die für das Azetidinderivat angegebenen Bedeutungen aufweisen, oder durch Cyclisieren eines Esters einer Verbindung der allgemeinen Formel

$$Hal—X—Y—\underset{\displaystyle Hal}{\underset{|}{CH}}—CO_2H \qquad , (III)$$

worin Hal, X und Y die vorstehend angegebenen Bedeutungen besitzen, in Gegenwart eines Amins $ANH_2$, worin A die vorstehend angegebene Bedeutung hat, und anschließendes Entfernen der Gruppe A aus dem Azetidinring; und im Falle, daß ein Azetidinderivat aus dem Verfahren (a) oder (b) resultiert und ein anderes Azetidinderivat benötigt wird, Umwandeln des erhaltenen Derivats in das gewünschte Derivat.

14. Ein Verfahren nach Anspruch 13, worin im Verfahren (a) eine Schutzgruppe A vorliegt, die eine Benzyl- oder eine $\alpha$-Phenylbenzylgruppe ist, welche Gruppe aus dem Azetidinring nach der Hydrolyse oder Alkoholyse der Cyanogruppe durch Hydrierung unter Anwendung von gasförmigem Wasserstoff und einem Katalysator abgespalten wird.

15. Ein Verfahren nach Anspruch 13, in welchem im Verfahren (b) eine Verbindung der Formel (II) durch Behandlung mit Bariumhydroxid cyclisiert wird.

16. Ein Verfahren nach Anspruch 13, in welchem im Verfahren (b) eine Verbindung der Formel (III) in Gegenwart von Benzylamin oder $\alpha$-Phenylbenzylamin cyclisiert wird.

17. Eine Verbindung nach Anspruch 11, wenn sie nach einem Verfahren gemäß einem der Ansprüche 13 bis 16 hergestellt ist.

**Patentansprüche für den Vertragsstaat: AT**

1. Ein Verfahren zur Sterilisation der männlichen Teile einer Pflanze, dadurch gekennzeichnet, daß es das Aufbringen eines Azetidinderivats auf die Pflanze umfaßt, welches Derivat eine Säure der allgemeinen Formel (I) oder ein Salz und/oder ein Ester, Amid, Alkylamid, Hydrazid oder Alkylhydrazid hievon ist:

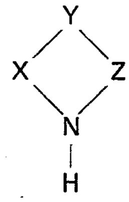

, (I)

worin

X eine der Gruppen $CH_2$, CHR oder $CR_2$ bedeutet;

Y eine der Gruppen CHR, $CR_2$ oder $CH.CO_2H$ bedeutet und

Z eine der Gruppen $CH_2$, CHR, $CR_2$ oder $CH.CO_2H$ darstellt;

der oder jeder Rest R unabhängig voneinander eine Alkyl-, Alkenyl- oder Cycloalkylgruppe oder eine Aryl- oder Aralkylgruppe bedeutet, die gewünschtenfalls am Arylkern durch eine oder mehrere gleiche oder verschiedene Substituenten, ausgewählt unter Halogenatomen, Alkylgruppen und Alkoxygruppen, substituiert sind;

und eines, aber nur eines der Symbole Y und Z eine $CH.CO_2H$-Gruppe darstellen muß.

2. Ein Verfahren nach Anspruch 1, worin der oder jeder Rest R unabhängig voneinander eine Alkyl- oder Alkenylgruppe mit bis zu 6 Kohlenstoffatomen, eine Cycloalkylgruppe mit 3 bis 6 Kohlenstoffatomen oder eine Phenyl- oder Benzylgruppe bedeutet, die gegebenenfalls durch eine oder mehrere gleiche oder verschiedene Substituenten, ausgewählt unter Chlor-, Brom- oder Fluoratomen, Alkylgruppen mit 1 bis 4 Kohlenstoffatomen oder Alkoxygruppen mit 1 bis 4 Kohlenstoffatomen, substituiert sind.

3. Ein Verfahren nach Anspruch 2, worin der oder jeder Rest R unabhängig voneinander eine Methyl- oder Ethylgruppe oder eine Phenylgruppe darstellt, die gegebenenfalls durch einen oder zwei Substituenten, ausgewählt unter Fluor- und Chloratomen und Methyl- und Methoxygruppen, substituiert ist.

4. Ein Verfahren nach einem der Ansprüche 1 bis 3, worin X für $CH_2$, Y für CHR oder $CH.CO_2H$ und Z für $CH_2$ oder $CH.CO_2H$ stehen.

5. Ein Verfahren nach einem der Ansprüche 1 bis 4, worin das Azetidinderivat die freie Säure der allgemeinen Formel (I) oder ein Hydrohalogenid- oder ein Alkalimetallsalz hievon ist; das Amid oder Hydrazid hievon ist, worin die Amid- oder Hydrazidgruppe durch ein oder zwei Alkylgruppen substituiert sein kann; ein Alkyl-, Alkenyl- oder Aralkylester ist; oder ein Hydrohalogenid des genannten Amids, Hydrazids oder Esters ist.

6. Ein Verfahren nach Anspruch 5, worin das Azetidinderivat die freie Säure der Formel (I), ein Hydrohalogenid oder ein Alkalimetallsalz hievon, das Hydrazid hievon, ein $C_{1-10}$-alkylester hievon oder ein Hydrohalogenid des genannten Hydrazids oder Esters ist.

7. Ein Verfahren nach Anspruch 1, worin das Azetidinderivat 3-Carboxyazetidin oder 2-Carboxy-3-methylazetidin ist.

8. Ein Verfahren zur Herstellung von $F_1$-Hybridsamen, welches ein kreuzweises Befruchten einer Pflanze, die nach einem Verfahren gemäß einem der Ansprüche 1 bis 7 behandelt worden ist, mit einer zweiten Pflanze eines unterschiedlichen Stammes umfaßt.

9. Eine männliche Sterilität in Pflanzen hervorrufende Zusammensetzung, welche ein Azetidinderivat gemäß Definition in einem der Ansprüche 1 bis 7, zusammen mit einem geeigneten Träger, umfaßt, mit der Maßgabe, daß dann, wenn das Azetidinderivat 3-Ethyl-2-carboxyazetidin ist und nur ein Träger vorliegt, dieser Träger nicht Ethanol oder Wasser ist, daß dann, wenn das Azetidinderivat 3-Carboxyazetidin ist und nur ein Träger vorliegt, dieser Träger nicht Wasser ist und daß dann, wenn das Azetidinderivat 3-Carboxyazetidin ist und nur zwei Träger vorliegen, diese Träger nicht Ethanol und Wasser sind.

10. Eine Zusammensetzung nach Anspruch 9, welche wenigstens zwei Träger umfaßt, wovon wenigstens einer ein oberflächenaktives Mittel ist.

11. Ein Verfahren zur Herstellung einer Verbindung gemäß Definition in Anspruch 1, worin X eine Gruppe CHR oder $CR_2$ bedeutet, wenn Y für $CH.C_2H_5$ oder $CH.CO_2H$ steht, welches umfaßt entweder

(a) Bereitung eines Azetidinderivates, worin Y eine $CH.CO_2H$-Gruppe bedeutet, durch Hydrolyse oder Alkoholyse der entsprechenden Cyanoverbindung, in welcher Verbindung das 1-Stickstoffatom des Ringes durch eine Gruppe A substituiert sein kann, welche eine Schutzgruppe ist, die von dem Azetidinring entfernt werden kann; und, falls erforderlich, Abtrennung der Schutzgruppe; oder

(b) Bereitung eines Azetidinderivats, in welchem Z eine $CH.CO_2H$-Gruppe darstellt, durch Cyclisieren einer Verbindung der allgemeinen Formel

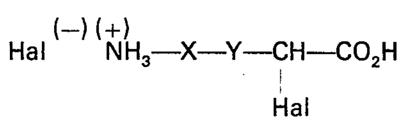

, (II)

worin jeder Rest Hal unabhängig voneinander ein Chlor- oder Bromatom darstellt und X und Y die für das Azetidinderivat angegebenen Bedeutungen aufweisen, oder durch Cyclisieren eines Esters einer Verbindung der allgemeinen Formel

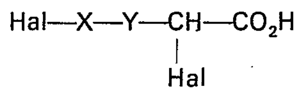
, (III)

worin Hal, X und Y die vorstehend angegebenen Bedeutungen besitzen, in Gegenwart eines Amins $ANH_2$, worin A die vorstehend angegebene Bedeutung hat, und anschließendes Entfernen der Gruppe A aus dem Azetidinring; und im Falle, daß ein Azetidinderivat aus dem Verfahren (a) oder (b) resultiert und ein anderes Azetidinderivat benötigt wird, Umwandeln des erhaltenen Derivats in das gewünschte Derivat.

12. Ein Verfahren nach Anspruch 11, worin im Verfahren (a) eine Schutzgruppe A vorliegt, die eine Benzyl- oder eine $\alpha$-Phenylbenzylgruppe ist, welche Gruppe aus dem Azetidinring nach der Hydrolyse oder Alkoholyse der Cyanogruppe durch Hydrierung unter Anwendung von gasförmigem Wasserstoff und einem Katalysator abgespalten wird.

13. Ein Verfahren nach Anspruch 11, in welchem im Verfahren (b) eine Verbindung der Formel (II) durch Behandlung mit Bariumhydroxid cyclisiert wird.

14. Ein Verfahren nach Anspruch 11, in welchem im Verfahren (b) eine Verbindung der Formel (III) in Gegenwart von Benzylamin oder $\alpha$-Phenylbenzylamin cyclisiert wird.

**Revendications pour les Etats contractants: BE CH DE FR GB IT NL SE**

1. Procédé de stérilisation des organes males d'une plante caractérisé en ce qu'il comprend l'application à la plante d'un dérivé d'azétidine qui est un acide de formule générale I ou un sel et/ou un ester, amide, alkylamide, hydrazide ou alkyhydrazide correspondant:

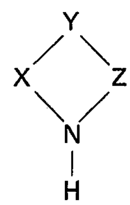
(I)

dans laquelle

X représente un des groupes $CH_2$, CHR, $CR_2$;

Y représente un des groupes CHR, $CR_2$ ou $CH.CO_2H$

Z représente un des groupes $CH_2$, CHR, $CR_2$ ou $CH.CO_2H$;

le symbole R, ou chacun des symboles R, représente indépendamment un groupe alkyle, alcényle, ou cycloalkyle ou un groupe aryle ou aralkyle éventuellement substitués sur le noyau aryle par un ou plusieurs substituants semblables ou différents choisis parmi les atomes d'halogène, les groupes alkyles et les groupes alcoxy;

et un, mais un seulement, de Y et Z doit représenter un groupe $CH.CO_{2H}$.

2. Un procédé comme revendiqué dans la revendication 1, dans lequel le symbole R, ou chacun des symboles R, représente indépendamment un groupe alkyle ou alcényle ayant jusqu'à 6 atomes de carbone, un groupe cycloalkyle ayant 3 à 6 atomes de carbone ou un groupe phényle ou benzyle éventuellement substitués par un ou plusieurs substituants semblables ou différents choisis parmi les atomes de chlore, de brome ou de fluore, les groupes alcoxy ayant 1 à 4 atomes de carbone ou les groupes alcoxy ayant 1 à 4 atomes de carbone.

3. Un procédé comme revendiqué dans la revendication 2, dans lequel le symbole R, ou chacun des symboles R, représente indépendamment un groupe méthyle ou éthyle ou un groupe phényle éventuellement substitué par un ou deux substituants choisis parmi les atomes de fluor et de chlore et les groupes méthyles et méthoxy.

4. Un procédé comme revendiqué dans l'une quelconque des revendications 1 à 3, dans lequel X représente $CH_2$, Y représente CHR ou $CH.CO_2H$ et Z représente $CH_2$ ou $CH.CO_{2H}$.

5. Un procédé comme revendiqué dans l'une quelconque des revendications 1 à 4, dans lequel le dérivé azétidine est l'acide libre de formule générale I ou un halogenhydrate ou un sel de métal alcalin correspondant, l'amide ou l'hydrazide correspondant où le groupe amide ou hydrazide peut être substitué par un ou deux groupes alkyles; un ester alkylique, alcénylique ou aralkylique; ou un halogénehydrate dudit amide, hydrazide ou ester.

6. Un procédé comme revendiqué dans la revendication 5, dans lequel le dérivé d'azétidine est l'acide libre de formule I, un halogénehydrate ou un sel de métal alcalin correspondant, l'hydrazide correspondant, un ester d'alkyle en C(1—10) correspondant ou un halogénehydrate dudit hydrazide ou ester.

7. Un procédé comme revendiqué dans la revendication 1, dans lequel le dérivé d'azétidine est la 3-carboxyazétidine ou la 2-carboxy-3-méthylazétidine.

8. Un procédé pour produire des semences d'hybrides F1 qui comprend une pollinisation croisée d'une plante que l'on a traitée selon un procédé comme revendiqué dans l'une quelconque des revendications 1 à 7, avec une seconde plante d'une lignée différente.

9. Composition produisant la stérilité mâle chez les plantes qui comprend un dérivé d'azétidine, comme défini dans l'une quelconque des revendications 1 à 7, avec un support approprié; sous réserve que, si le dérivé d'azétidine est la 3-éthyl-2-carboxyazétidine et un seul support est présent, le support n'est ni l'éthanol, ni l'eau, si le dérivé d'azétidine est la 2-carboxyazétidine et un seul support est présent, ce support n'est pas l'eau et si le dérivé d'azétidine est la 2-carboxyazétidine et deux supports seulement sont présents, ces supports ne sont ni l'éthanol, ni l'eau.

10. Une composition comme revendiquée dans la revendication 9, qui comprend au moins deux supports dont au moins un est un agent tension-actif.

11. Un dérivé d'azétidine comme défini dans l'une quelconque des revendications 1 à 3, sous réserve que si Y représente $CH.C_2H_5$ ou $CH.CO_2H$, X doit représenter un groupe CHR ou $CR_2$.

12. 3-méthyl-2-carboxyazétidine.

13. Un procédé pour la préparation d'un composé comme revendiqué dans la revendication 11, qui comprend soit

(a) la préparation d'un dérivé d'azétidine dans lequel X représente un groupe $CH.CO_2H$, par hydrolyse ou alcoolyse du composé cyano correspondant, dans lequel composé l'atome d'azote en position 1 du cycle peut être substitué par un groupe A qui est un groupe protecteur pouvant être éliminé du cycle azétidine; et, s'il est nécessaire, l'élimination du groupe protecteur; soit

(b) la préparation d'un dérivé d'azétidine dans lequel Z représente un groupe $CH.CO_2H$ par cyclisation d'un composé de formule générale

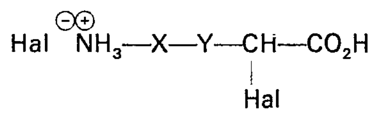

(II)

dans laquelle chaque Hal représente indépendamment un atome de chlore ou de brome et X et Y ont les significations indiquées pour le dérivé d'azétidine, ou par cyclisation d'un ester d'un composé de formule générale

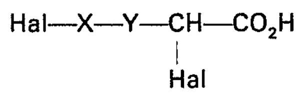

(III)

dans laquelle Hal, X et Y ont les significations indiquées ci-dessus, en présence d'une amine $ANH_2$ où A a la signification indiquée ci-dessus, puis l'élimination du groupe A du cycle azétidine; et dans les cas où on obtient par le procédé (a) ou (b) un dérivé d'azétidine et qu'on désire un dérivé d'azétidine différent, la conversion du dérivé obtenu en le dérivé désiré.

14. Un procédé comme revendiqué dans la revendication 13, dans lequel dans le procédé (a), un groupe protecteur A qui est un groupe benzyle ou un groupe $\alpha$-phénylbenzyle est présent, ce groupe étant éliminé du cycle azétidine après hydrolyse ou alcoolyse du groupe cyano, par hydrogénation avec de l'hydrogène gazeux et un catalyseur.

15. Un procédé comme revendiqué dans la revendication 13, dans lequel, dans le procédé (b), un composé de formule II est cyclisé par traitement avec l'hydroxyde de baryum.

16. Un procédé comme revendiqué dans la revendication 13, dans lequel, dans le procédé (b), un composé de formule III est cyclisé en présence de benzylamine ou d'$\alpha$-phénylbenzylamine.

17. Un composé comme revendiqué dans la revendication 11, que l'on a préparé selon un procédé comme revendiqué dans l'une quelconque des revendications 13 à 16.

**Revendications pour l'Etat contractant: AT**

1. Procédé de stérilisation des organes mâles d'une plante, caractérisé en ce qu'il comprend l'application à la plante d'un dérivé d'azétidine qui est un acide de formule générale I ou un sel et/ou un ester, amide, alkylamide, hydrazide ou alkylhydrazide correspondant:

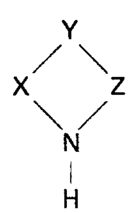

(I)

dans laquelle

X représente un des groupes $CH_2$, CHR ou $CR_2$;

Y représente un des groupes CHR, $CR_2$ ou $CH.CO_2H$;

Z représente un des groupes $CH_2$, CHR, $CR_2$ ou $CH.CO_2H$;

le symbole R, ou chacun des symboles R, représente indépendamment un groupe alkyle, alcényle ou cycloalkyle ou un groupe aryle ou aralkyle éventuellement substitués sur le noyau aryle par un ou plusieurs substituants semblables ou différents choisis parmi les atomes d'halogène, les groupes alkyles et les groupes alcoxy;

et un, mais un seulement, de Y et Z doit représenter un groupe $CH.CO_2H$.

2. Un procédé comme revendiqué dans la revendication 1, dans lequel le symbole R, ou chacun des symboles R, représente indépendamment un groupe alkyle ou alcényle ayant jusqu'à 6 atomes de carbone, un groupe cycloalkyle ayant 3 à 6 atomes de carbone ou un groupe phényle ou benzyle éventuellement substitués par un ou plusieurs substituants semblables ou différents choisis parmi les atomes de chlore, de brome ou de fluor, les groupes alkyles ayant 1 à 4 atomes de carbone ou les groupes alcoxy ayant 1 à 4 atomes de carbone.

3. Un procédé comme revendiqué dans la revendication 2, dans lequel le symbole R, ou chacun des symboles R, représente indépendamment un groupe méthyle ou éthyle ou un groupe phényle éventuellement substitué par un ou deux substituants choisis parmi les atomes de fluor et de chlore et les groupes méthyles et méthoxy.

4. Un procédé comme revendiqué dans l'une quelconque des revendications 1 à 3, dans lequel X représente $CH_2$, Y représente CHR ou $CH.CO_2H$ et Z représente $CH_2$ ou $CH.CO_2H$.

5. Un procédé comme revendiqué dans l'une quelconque des revendications 1 à 4, dans lequel le dérivé d'azétidine est l'acide libre de formule générale I ou un halogénehydrate ou un sel de métal alcalin correspondant; l'amide ou l'hydrazide correspondant ou le groupe amide ou hydrazide peut être substitué par un ou deux groupes alkyles; un ester alkylique, alcénylique ou aralkylique; ou un halogénehydrate dudit amide, hydrazide ou ester.

6. Un procédé comme revendiqué dans la revendication 5, dans lequel le dérivé d'azétidine est l'acide libre de formule I, un halogénehydrate ou un sel de métal alcalin correspondant, l'hydrazide correspondant, un ester d'alkyle en C(1—10) correspondant ou un halogénehydrate dudit hydrazide ou ester.

7. Un procédé comme revendiqué dans la revendication 1, dans lequel le dérivé d'azétidine est la 3-carboxyazétidine ou la 2-carboxy-3-méthylazétidine.

8. Un procédé pour produire des semences d'hybrides F1 qui comprend la pollinisation croisée d'une plante que l'on a traitée selon un procédé comme revendiqué dans l'une quelconque des revendications 1 à 7, avec une seconde plante d'une lignée différente.

9. Composition produisant la stérilité mâle chez les plantes qui comprend un dérivé d'azétidine comme défini dans l'une quelconque des revendications 1 à 7, avec un support approprié; sous réserve que, si le dérivé d'azétidine est la 3-éthyl-2-carboxyazétidine et un seul support est présent, ce support n'est ni l'éthanol ni l'eau, si le dérivé d'azétidine est la 3-carboxyazétidine et un seul support est présent, ce support n'est pas l'eau et si le dérivé d'azétidine est la 3-carboxyazétidine et deux supports seulement sont présents, ces supports ne sont ni l'éthanol, ni l'eau.

10. Un composition comme revendiquée dans la revendication 9, qui comprend au moins deux supports dont au moins un est un agent tensio-actif.

11. Un procédé pour la préparation d'un composé comme défini dans la revendication 1, où X représente un groupe CHR ou $CR_2$ et Y représente $CH.C_3Hs$ ou $CH.CO_2H$, qui comprend soit

(a) la préparation d'un dérivé d'azétidine dans lequel X représente un groupe $CH.CO_2H$ par hydrolyse ou alcoolyse du composé cyano correspondant, dans lequel composé l'atome d'azote en position 1 du cycle peut être substitué par un groupe A qui est un groupe protecteur pouvant être éliminé du cycle azétidine; et, s'il est nécessaire, l'élimination du groupe protecteur; soit

(b) la préparation d'un dérivé d'azétidine dans lequel Z représente un groupe $CH.CO_2H$ par cyclisation d'un composé de formule générale

$$\overset{-}{Hal}\ \overset{+}{N}H_3—X—Y—\underset{\displaystyle Hal}{\underset{|}{CH}}—CO_2H \qquad (II)$$

dans laquelle chaque Hal représente indépendamment un atome de chlore ou de brome et X et Y ont les significations indiquées pour le dérivé d'azétidine, ou par cyclisation d'un ester d'un composé de formule générale:

$$Hal—X—Y—\underset{\displaystyle Hal}{\underset{|}{CH}}—CO_2H \qquad (III)$$

dans laquelle Hal, X et Y ont les significations indiquées ci-dessus, en présence d'une amine $ANH_2$ où A

a la signification indiquée ci-dessus, puis l'élimination du groupe A du cycle azétidine; et, dans les cas où on obtient par le procédé (a) ou (b) un dérivé d'azétidine et qu'on désire un dérivé d'azétidine différent, la conversion du dérivé obtenu en le dfrivé désiré.

12. Un procédé comme revendiqué dans la revendication 11, dans lequel dans le procédé (a), un groupe protecteur A qui est un groupe benzyle ou un groupe $\alpha$-phénylbenzyle est présent, ce groupe étant éliminé du cycle azétidine après hydrolye ou alcoolyse du groupe cyano, par hydrogénation avec de l'hydrogène gazeux et un catalyseur.

13. Un procédé comme revendiqué dans la revendication 11, dans lequel, dans le procédé (b), un composé de formule II est cyclisé par traitement avec l'hydroxyde de baryum.

14. Un procédé comme revendiqué dans la revendication 11, dans lequel, dans le procédé (b), un composé de formule III est cyclisé en présence de benzylamine ou d'$\alpha$-phénylbenzylamine.